Europäisches Patentamt

European Patent Office

Office européen des brevets

(11) Veröffentlichungsnummer: **0 387 753**
**A1**

(12) **EUROPÄISCHE PATENTANMELDUNG**

(21) Anmeldenummer: 90104637.5

(22) Anmeldetag: **12.03.90**

(51) Int. Cl.5: **A61B 17/22, C03B 37/15, C03C 25/00, G02B 6/26**

(30) Priorität: **17.03.89 DE 8903333 U**

(43) Veröffentlichungstag der Anmeldung:
**19.09.90 Patentblatt 90/38**

(84) Benannte Vertragsstaaten:
**AT CH DE FR GB IT LI NL SE**

(71) Anmelder: **Schott Glaswerke**
**Hattenbergstrasse 10**
**D-6500 Mainz(DE)**

(84) **CH DE FR GB IT LI NL SE AT**

Anmelder: **Carl-Zeiss-Stiftung trading as**
**SCHOTT GLASWERKE**
**Hattenbergstrasse 10**
**D-6500 Mainz 1(DE)**

(84) **GB**

(72) Erfinder: **Müller, Gerhard, Prof., Dr.**
**An der Rehwiese 8**
**D-1000 Berlin 38(DE)**
Erfinder: **Kar, Hasan, Dipl.-Phys.**
**Schildhornstrasse 22**
**D-1000 Berlin 41(DE)**
Erfinder: **Dörschel, Klaus, Dr.**
**Himbeersteig 16**
**D-1000 Berlin 38(DE)**
Erfinder: **Schönborn, Karl-Heinz, Dr.**
**Konrad-Adenauer-Strasse 27**
**D-6500 Mainz 41(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) Verfahren und Vorrichtung zum Schutz der proximalen Einkoppelseiten von Laserkathetern.

(57)
I. In einem Verfahren zum Schutz der einkoppelseitigen Enden von mit kurzgepulster Laserstrahlung beaufschlagten Laserkathetern werden

a) die einzelnen Lichtleitfaser (13) einkoppelseitig in hexagonal dichtester Packung angeordnet,

b) an den an der äußeren Zirkumferenz der Einkoppelvorrichtung anschließenden Querschnittsbereich ablationsresistente Mittel (14) angebracht und

c) die einzelnen Fasern und die ablationsresistenten Mittel über Fügeverfahren stoff- und/oder kraftschlüssig miteinander verbunden.

II. Vorrichtungen zum Schutz der einkoppelseitigen Enden von mit kurzgepulster Laserstrahlung beaufschlagten Laserkathetern sind nach den oben beschriebenen Verfahren hergestellt worden, wobei die ablationsresistenten Mittel aus dem gleichen Material wie die Lichtleitfasern, aus Silikat oder aus PTFE (14) sind.

# Fig.5

## Verfahren und Vorrichtung zum Schutz der proximalen Einkoppelseiten von Laserkathetern

Die Erfindung betrifft ein Verfahren zum Schutz der einkoppelseitigen Enden von Laserkathetern für die Fotoablation von Ablagerungen in Gefäßsystemen des menschlichen Körpers.

An Laserkatheter, die zur Rekanalisation röhrenförmiger Hohlsysteme, insbesondere im medizinischem Bereich, Anwendung finden, werden bei den hierzu erforderlichen Schwellenenergiedichten erhebliche Anforderungen an Belastbarkeit und Haltbarkeit des Systems gestellt. Eine besondere Schwachstelle, die bei den üblichen Einsatzbedingungen der Laserstrahlung sehr bald zur Selbstzerstörung des Laserkatheters und damit zur Verminderung der Standzeit führt, bildet die Einkopplung der hochenergetischen, gepulsten Laserstrahlung am proximalen Ende des Katheters.

Für Fotoablationszwecke werden einkoppelseitig Energiedichten von 20 J/cm$^2$ benötigt. Im Spektralbereich zwischen 240 und 3.000 nm müssen kurze energiereiche Laserimpulse in eine oder mehrere Lichtleitfasern eingekoppelt werden. Die Schwellenenergiedichten, die für Fotoablationen zum Zwecke der Gefäßrekanilisation, insbesondere auch bei kalzifizierten Verschlüssen, aufgebracht werden müssen, sind hoch genug, um organisches Material abzutragen.

Da sich in der Regel wegen der beim arbeiten mit Laserkathetern unvermeidbaren, teils ruckartigen Laserstrahls auch die Einkopplungsfläche des ein-oder mehrfasrigen Laserkatheters verändern kann, läßt sich während des Betriebs eine Überstrahlung über den effektiven Querschnitt der Faser oder des Faserbündels an den Stirnseiten der Einkoppelfläche nicht dauerhaft vermeiden. Darüber hinaus läßt sich der Strahlquerschnitt nicht ohne weiteres dem Querschnitt eines Mehrfaserkatheters anpassen. Auch hier ist eine teilweise Überstrahlung umungänglich. Die für die Halterung der Lichtfasern nach dem Stand der Technik üblicherweise verwendeten Materialien halten dabei der auffallenden Energiedichte nicht stand. Die Folge ist eine Ablation des Haltematerials auf der Einkoppelseite, was eine frühzeitige Zerstörung des Katheters zur Folge hat.

Bei den verwendeten Pulsenergien führt die Ablation des Haltematerials bei Verwendung metallischer Stoffe zur Ablagerung metallischer Partikel auf der Einkoppelfläche der Fasern, und dadurch bedingt zur Zerstörung der Faser. Die Verwendung normaler Kunststoffe führt zu einer Zerstörung dieser Kunststoffe, einem Verlust der Fixierung und einer Faserzerstörung als Folge der auf der Faserendfläche abgelagerten Zersetzungsprodukte des Kunststoffs. Dies kann während des praktischen Einsatzes erhebliche Risiken für den Erfolg einer Rekanalisation mit sich bringen.

Der Erfindung liegt daher die Aufgabe zugrunde, eine Vorrichtung und Verfahren zum Schutz der einkoppelseitigen Enden von Laserkathetern zur Verfügung zu stellen, die es ermöglichen, die eingangs aufgezeigten Beeinträchtigungen beim Betrieb von Laserkathetern zurückzudrängen und somit die Standzeit von Laserkathetern erheblich zu erhöhen.

Diese Aufgabe wird erfindungsgemäß mit einem Verfahren nach den kennzeichnenden Merkmalen des Anspruchs 1 und mit einer Vorrichtung nach den kennzeichnenden Merkmalen des Anspruchs 8 gelöst.

Für die Einkopplungsvorrichtung am proximalen Ende ergeben sich mehrere Möglichkeiten unter Verwendung physikalischer und/oder laserlichtresistenter Bindemittel. Die Anordnung der Quarz/Quarz-Fasern in Mehrfaser-Ringkathetern werden an der Einkoppelstelle aus Gründen des maximalen geometrischen Koppelwirkungsgrades in hexagonal dichtester Packung angeordnet. Die Einkopplung des Laserlichts in den Mehrfaser-Ringkatheter erfolgt zweckmäßigerweise über Steckverbindungen. Als Steckergrundkörper kann in den meisten Fällen eine Metallhülse verwendet werden, in welche die Lichtleitfasern mittels Stoff-und/oder Kraftschluß eingelassen sind. Die Lichtleitfasern werden dabei im Bereich der Fügestelle von ihrer Kunststoffummantelung befreit.

Eine Möglichkeit besteht darin, die Quarz/Quarz-Faser in hexagonal dichtester Packung in ein dünnes Quarzrohr bis zur Erweichungstemperatur zu erhitzen und unter leichtem axialen Zug miteinander zu verschmelzen. Anschließend wird in der Mitte der Schmelzstelle das Bündel aufgetrennt und die Stirnseite optisch poliert. Das auf diese Weise entstandene miteinander verschmolzene Quarzendstück hat die gleichen optischen Eigenschaften für die Einzelfasern und kann in einfacher Weise in eine metallische Hülse als Stecker aufgenommen werden.

In einer zweiten Möglichkeit werden die Faserbündel in hexagonal dichtester Packung thermisch in eine Metallfassung eingeschrumpft, wobei bevorzugt Aluminium eingesetzt wird. Zum Schutz der Metalloberfläche wird diese entweder mit Polymethylsiloxan (Silikonkautschuk) oder einem Silikat beschichtet. In einer bevorzugten Ausführungsform steht das Faserbündel etwa um einen mm über das stirnseitige Ende der Metallfassung hinaus.

Ein weiterer Lösungsweg besteht darin, die Quarz/Quarz-Fasern eines Mehrfaser-Ringkatheters in hexagonal dichtester Packung unter Verwendung von Silikat als Fügemittel in eine metallische Hülse einzubetten. In einer bevorzugten Ausführungsform wird dabei Natriumhydrosilikat (Wasserglas) eingesetzt. Anschließend wird wiederum das überstehende Faserbündel randständig zum Stecker abgetrennt und die Endfläche optisch poliert.

Eine wesentliche Erhöhung der Standzeit konnte überraschend errreicht werden, wenn die Fassung der Fasern im aktiven Strahlbereich unter Verwendung von Polytetrafluorethylen (PTFE) erfolgte. Dieses Material besitzt besonders im für medizinische Anwendungen kritischen UV-Wellenlängenbereich von 300 bis 350 nm eine gute Ablationsfestigkeit. Die PTFE-Fassungen werden ihrerseits wieder in metallischen Hülsen aufgenommen. Im Falle von Mehrfaser-Ringkathetern werden die Einzelfasern in hexagonal dichtester Packung mittels eines PTFE-Schrumpfschlauchs zusammengehalten, während die Erweiterung des stirnseitigen ablationsresistenten Querschnittsbereichs über PTFE-Paßteile in Form eines Ringes erfolgt.

Reicht der PTFE-Schrumpfschlauch bis zur Einkoppelfläche des Faserbündels, so kann es vorkommen, daß bei Laserbestrahlung das PTFE über die Endflächen kriecht und somit die Laserlichteinkopplung stört. Dies kann umgangen werden, wenn die Faserenden nicht völlig bis zur Einkoppelfläche von PTFE-Schrumpfschlauch umschlossen werden, so daß die Fasern eine hinreichende Länge freistehen. In diesem Falle kann jedoch das freistehende Faserbündel an seiner Stirnseite nachträglich nicht optisch poliert werden. Dieser Nachteil kann dadurch umgangen werden, daß eine Außenlage von zur Übertragung nicht benutzten kurzen Faserstücken mit eingeschrumpft wird. In diesem Falle kann der Schrumpfschlauch bis zum Faserende reichen, wobei er so viel mechanische Stabilität erreicht, daß eine nachträgliche Politur der Endfläche des gesamten Faserbündels möglich ist.

Im folgenden werden anhand der beigefügten schematischen Zeichnungen bevorzugte Ausführungsformen der erfindungsgemäßen Vorrichtungen für das einkopplungsseitige Ende des Laserkatheters im einzelnen noch näher erläutert.

Es zeigen:

Figur 1 einen schematischen Längsschnitt durch einen Einkoppelstecker, in dem die Lichtleitfasern in einem dünnen Quarzrohr aufgenommen und zusammen mit diesem verschmolzen werden,

Figur 2 einen schematischen Längsschnitt durch einen Einkoppelstecker, in dem ein Faserbündel thermisch in eine eingangsseitig beschichtete Metallhülse eingeschrumpft wurde,

Figur 3 einen schematischen Längsschnitt durch einen Einkoppelstecker, in dem Lichtleitfaserbündel in einer metallischen Hülse mit Silikat-Kleber eingebettet wurden,

Figur 4 einen schematischen Längsschnitt durch einen Einkoppelstecker, in dem eine Quarz/Quarz-Faser im aktiven Strahlbereich in ein PTFE-Teil eingeformt ist, welches seinerseits in einer Metallhülse verankert ist,

Figur 5 einen schematischen Längsschnitt durch einen Einkoppelstecker, in dem ein von PTFE-Schrumpfschlauch zusammengehaltenes Faserbündel in einen PTFE-Teil eingefaßt ist, welcher seinerseits in einer Metallhülse verankert ist.

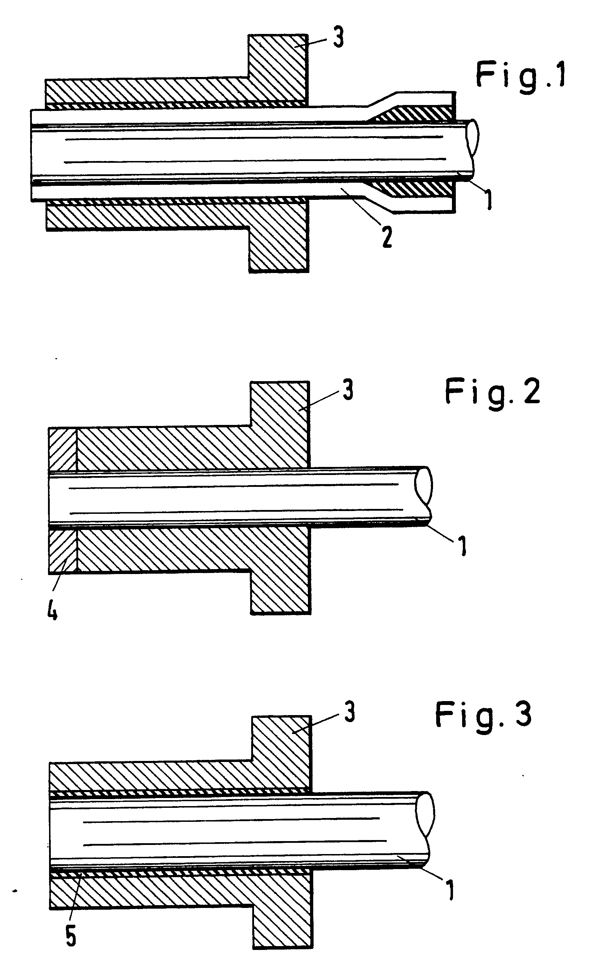

In Figur 1 ist ein Längsschnitt eines Einkoppelsteckers wiedergegeben. Die Quarz/Quarz-Faserbündel in hexagonal dichtester Packung 1 werden in einem Quarzrohr 2 aufgenommen. Dieses wird seinerseits in einer metallischen Hülse 3 gehalten.

Eine andere Ausführungsform für einen Einkoppelstecker wird in Figur 2 gezeigt. Die in hexagonal dichtester Packung gefaßten Lichtleitfasern 1 sind direkt in eine Metallhülse 3, vorzugsweise Aluminium, eingeschrumpft. Die Metallhülse ist zum Schutz vor Laserlicht am eintrittsseitigen Ende mit einer Schutzschicht 4, vorzugsweise aus Polymethylsiloxan oder Silikat überzogen.

In einer dritten Ausführungsforms wird, wie in Figur 3 gezeigt, die hexagonal dichteste Packung der Lichtleitfasern 1 über ein Silikatbett 5 in der Metallfassung 3 verankert, so daß an der eintrittsseitigen Stirnfläche die gegen das Laserlicht unempfindliche Querschnittsfläche des Lichtleiterbündels um die Stirnfläche des Silikatbettes 5 vergrößert wird.

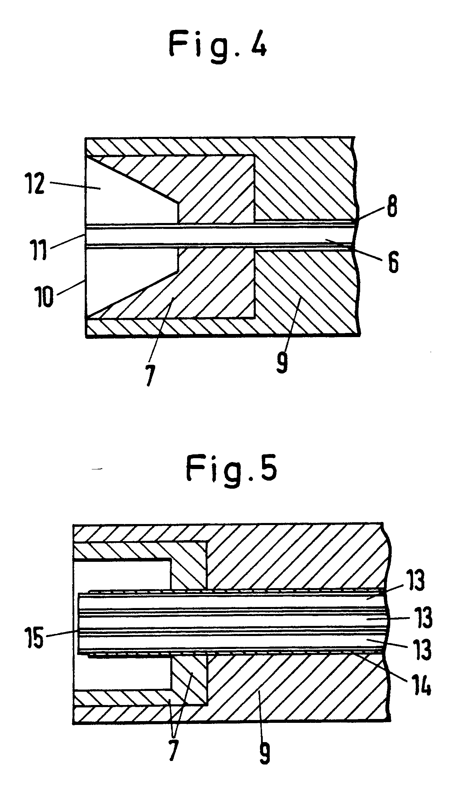

In Figur 4 wird die Fassung einer einzelnen Quarz/Quarz-Faser 6 in einem Steckerinnenteil im Längsschnitt gezeigt. Die Quarz/Quarz-Faser 6 wird am koppelseitigen Ende des Steckers von einem PTFE-Teil in einer Bohrung gehalten, wobei sie längs dieses PTFE-Teils 7 von ihrer Kunststoffummantelung 8 befreit ist. Aus Gründen mechanischer Stabilität wird das PTFE-Teil 7 in einem metallischen Steckergrundkörper 9 gehalten. Die Faser 6 steht bis zur mechanischen Bezugsfläche 10 des Steckergrundkörpers 9 über. Die Möglichkeit einer ablationsbedingten Ablagerung auf der Faserendfläche 11 wird dabei durch die Aussparung 12 im PTFE-Halter 7 auf ein Minimum reduziert.

Bei Verwendung von Einkoppelsteckern von Mehrfaserringkathetern, wie in Figur 5 wiedergegeben, werden die einzelnen Fasern 13 in hexagonal dichtester Packung vorteilhafterweise von einem PTFE-Schrumpfschlauch 14 eingeschlossen. Einkoppelseitig werden die Fasern 13 mitsamt Schrumpfschlauch 14 wieder in einer Bohrung eines PTFE-Paßteils 7 gehalten, das seinerseits wieder in ein Steckergrundgehäuse 9 eingelassen ist. Der Schrumpfschlauch endet vor der Einkoppelfläche 15 des Faserbündels 13.

**Ansprüche**

1. Verfahren zum Schutz der einkoppelseitigen Enden von mit kurzgepulster Laserstrahlung beaufschlagten Laserkathetern für die Fotoablation von Ablagerungen im Gefäßsystem des menschlichen Körpers, dadurch gekennzeichnet, daß

a) zur Erreichung eines möglichst hohen Koppelwirkungsgrades die einzelnen Lichtleitfasern des Laserkatheters einkoppelseitig von ihrer Kunststoffummantelung befreit und in hexagonal dichtester Packung angeordnet werden,

b) an der äußeren Zirkumferenz der Einkoppelfläche zum Schutz des daran anschließenden Querschnittsbereichs der Einkoppelvorrichtung vor überstreichendem kurzgepulstem Laserlicht ablationsresistente Mittel angebracht werden und

c) die einzelnen Fasern und die ablationsresistenten Mittel über Fügeverfahren stoff- und/oder kraftschlüssig miteinander verbunden werden.

2. Verfahren nach Anspruch 1, dadurch gekennzeichnet, daß ein dünnes Rohr von gleichem Material wie die Lichtleiter und die hexagonal dichtest gepackten Einzelfasern miteinander verschmolzen werden und anschließend die Einkoppelstirnfläche poliert wird.

3. Verfahren nach Anspruch 1, dadurch gekennzeichnet, daß die hexagonal dichteste Packung des Faserbündels in eine Metallfassung, vorzugsweise aus Aluminium, eingeschrumpft wird und anschließend zum Schutz der Stirnseite der Metallfassung diese mit einer Schicht aus Polymethylsiloxan oder Silikat versehen wird.

4. Verfahren nach Anspruch 1, dadurch gekennzeichnet, daß die hexagonal dichteste Packung des Faserbündels in einer metallischen Hülse in Silikat, vorzugsweise Wasserglas, stoffschlüssig eingebettet wird und das überstehende Faserbündel randständig zum proximalen Ende der metallischen Hülse hin abgetrennt und anschließend an der Stirnfläche optisch poliert wird.

5. Verfahren nach Anspruch 1, dadurch gekennzeichnet, daß die hexagonal dichteste Packung des Faserbündels in eine oder mehrere Lagen von PTFE-Schrumpfschlauch kraftschlüssig eingebettet wird und sodann das Faserbündel mit umgebendem Schrumpfschlauch zum proximalem Ende hin in ein PTFE-Paßteil aufgenommen wird, das dann kraftschlüssig in einem Steckergrundkörper verankert wird.

6. Verfahren nach Anspruch 5, dadurch gekennzeichnet, daß die hexagonal dichteste Packung des Faserbündels in eine oder mehrere Lagen von PTFE-Schrumpfschlauch kraftschlüssig so eingebettet wird, daß der PTFE-Schrumpfschlauch ein kurzes Stück vor der Faserendfläche endet.

7. Verfahren nach den Ansprüchen 1 und 5, dadurch gekennzeichnet, daß zwischen das Faserbündel und den Schrumpfschlauch zum Schutz der aktiven Fasern eine Außenlage nicht benutzter Faserstücke mit eingeschrumpft wird wobei der Schrumpfschlauch bis zum Faserende aufgebracht und anschließend das Faserende optisch poliert wird.

8. Vorrichtung zum Schutz der einkoppelseitigen Enden von mit kurzgepulster Laserstrahlung beaufschlagten Laserkathetern, dadurch gekennzeichnet, daß

a) die einzelnen Lichtleitfasern (1,13) einkoppelseitig in hexagonal dichtester Packung angeordnet sind,

b) der an die äußere Zirkumferenz der Einkoppelfläche unmittelbar anschließende Querschnittsbereich der Einkoppelvorrichtung zum Schutz vor überstreichendem Laserlicht mit ablationsresistenten Mitteln besetzt ist und

c) die einzelnen Fasern (1,13) und die ablationsresistenten Mitteln stoff- und/oder kraftschlüssig miteinander verbunden sind.

9. Vorrichtung nach Anspruch 8, dadurch gekennzeichnet, daß das ablationsresistente Mittel ein dünnes Rohr (2) aus gleichem Material wie der Lichtleiter (1) ist.

10. Vorrichtung nach Anspruch 8 und 9, dadurch gekennzeichnet, daß das Rohr (2) und die Lichtleitfasern (1) miteinander verschmolzen sind.

11. Vorrichtung nach Anspruch 8, dadurch gekennzeichnet, daß das ablationsresistente Mittel ein Polymethylsiloxan oder ein Silikat ist.

12. Vorrichtung nach Anspruch 8, dadurch gekennzeichnet, daß das ablationsresistente Mittel ein hochtemperaturfester Silikatbinder, vorzugsweise Wasserglas, ist.

13. Vorrichtung nach Anspruch 8, dadurch gekennzeichnet, daß das ablationsresistente Mittel Polytetrafluorethylen (PTFE) ist.

14. Vorrichtung nach Anspruch 8 und 13, dadurch gekennzeichnet, daß eine Lichtleitfaser (6) oder ein Faserbündel (1,13) an ihrem proximalen Ende kraftschlüssig von einem PTFE-Teil (7) gehalten werden.

15. Vorrichtung nach Anspruch 14, dadurch gekennzeichnet, daß ein lichtleitfaserhaltender PTFE-Teil (7) in einer metallischen Steckvorrichtung (9) so angeordnet ist, daß die Faserendfläche (11,15) freisteht.

16. Vorrichtung nach Anspruch 8 und 14, dadurch gekennzeichnet, daß ein oder mehrere Lagen PTFE-Schrumpfschlauch (14) die hexagonal dichteste Packung des Faserbündels kraftschlüssig zusammenhält und diese Packung in einer Steckvorrichtung von einem PTFE-Ring (7) gehalten wird.

17. Vorrichtung nach Anspruch 16, dadurch gekennzeichnet, daß der PTFE-Schrumpfschlauch (14) ein kurzes Stück vor der Faserendfläche endet.

18. Vorrichtung nach Anspruch 8, dadurch gekennzeichnet, daß das ablationsresistente Mittel aus einer Außenlage von nicht zur Laserlicht-Übertragung benutzten kurzen Faserstücken besteht.

19. Vorrichtung nach Anspruch 16 und 18, dadurch gekennzeichnet, daß zum Schutz der aktiven Fasern eine Außenlage nicht benutzter Faserstücke mit eingeschrumpft ist und der Schrumpfschlauch bis zum Faserende reicht.

Fig.1

3

1

2

Fig.2

3

1

4

Fig.3

3

1

5

# Fig. 4

# Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 698 034 (SEVERIJNS et al.) * Spalte 6, Zeile 53 – Spalte 7, Zeile 23; Figur 3C * --- | 1,2,8,9,10 | A 61 B 17/22 C 03 B 37/15 C 03 C 25/00 G 02 B 6/26 |
| Y | US-A-4 770 486 (WANG et al.) * Spalte 3, Zeile 57 – Spalte 4, Zeile 3; Figur 3 * --- | 1,2,8,9,10 | |
| A | EP-A-0 155 051 (PHILIPS) * Anspruch 1 * --- | 3,11 | |
| A | JP-A-61 146 734 (AGENCY OF IND. SCIENCE & TECHNOL.) * Zusammenfassung * --- | 4,11,12 | |
| A | JP-A-61 208 008 (KOMATSU) * Zusammenfassung * --- | 5,13 | |
| A | US-A-4 652 288 (SAITO) * Anspruch 1; Figur 16 * --- | 1,8 | RECHERCHIERTE SACHGEBIETE (Int. Cl.5) |
| A | JP-A-5 717 903 (OLYMPUS) * Zusammenfassung * ----- | 1,8 | A 61 B C 03 B C 03 C G 02 B |

Der vorliegende Recherchenbericht wurde für alle Patentansprüche erstellt

| Recherchenort | Abschlußdatum der Recherche | Prüfer |
|---|---|---|
| DEN HAAG | 19-06-1990 | MOERS R.J. |

EPO FORM 1503 03.82 (P0403)